# FASCICULE DE BREVET EUROPEEN

(11) **EP 2 478 085 B1**
(45) Date de publication et mention de la délivrance du brevet: **29.04.2015**
(21) Numéro de dépôt: 10762719.2
(22) Date de dépôt: 10.09.2010
(51) Int. Cl.: C12M 1/18, B01L 3/00

(54) **MICRO-PLAQUE**
MIKROPLATTE
MICROPLATE

(30) Priorité: 14.09.2009 FR 0956292
(43) Date de publication de la demande: 25.07.2012
(73) Titulaire: Pierre Fabre Médicament, 92100 Boulogne (FR)
(72) Inventeur: SAMSON, Arnaud, F-31280 Dremil-Lafage (FR)
(74) Mandataire: Regimbeau
(86) Numéro de dépôt international: PCT/FR2010/000615
(87) Numéro de publication internationale: WO 2011/030018

(56) Documents cités:
- WO-A1-2006/005811
- WO-A2-2008/111035
- US-A- 5 587 321
- US-A- 5 908 776
- US-B1- 7 208 125

## Description

La présente invention concerne le domaine des dispositifs de microtitration. Plus particulièrement, l'invention a pour objet une micro-plaque, ou plaque de microtitration, présentant une invagination consistant en une rigole périphérique continue permettant d'y adjoindre, en une seule étape de distribution, un liquide faisant office de « rideau d'évaporation ».

Il est aujourd'hui reconnu par les divers utilisateurs que les micro-plaques utilisées en laboratoire ne donnent pas entière satisfaction en ce sens qu'elles peuvent être à l'origine d'erreurs au niveau de l'analyse des échantillons. De telles erreurs peuvent être notamment dues à un problème en terme de constitution de la plaque. Un des problèmes majeure réside dans le fait qu'il s'opère au sein d'une micro-plaque recouverclée un gradient d'évaporation entre les puits périphériques et les puits centraux. Ce gradient d'évaporation observé dans les puits de la micro-plaque est le fruit d'un gradient thermique et/ou hydrique entre l'environnement extérieur et l'intérieur de la plaque recouverclée, pouvant être le résultat d'une longue période d'incubation de la plaque, et pouvant alors fausser les résultats des analyses effectuées. En outre, il est à noter que les températures pour la réalisation d'analyses biologiques ou biochimiques peuvent être proches des températures de vaporisation de certains constituants des échantillons. Cela implique donc la vaporisation, c'est-à-dire le passage de l'état liquide à l'état gazeux de certains des solvants contenant les échantillons ou bien des échantillons eux-mêmes. Les puits victimes de ce gradient d'évaporation sont principalement les puits périphériques. Ils sont en effet soumis plus facilement aux fortes températures et aux variations d'hygrométrie que les puits du centre de la plaque. En effet, leurs parois atteignent plus rapidement les températures du milieu de conservation des échantillons contenus dans ces micro-plaques et ils se trouvent plus près de la zone de ventilation entre le bord externe de la plaque et le couvercle, ce qui aboutit à l'évaporation partielle et non homogène de certains constituants. Ce phénomène d'évaporation observé dans les puits périphériques, phénomène également connu sous le nom d'« effet de bords », ou encore « edge effect », peut fausser les analyses et la lecture des résultats.

Le brevet US 5,587,321 se propose de résoudre les problèmes d'évaporation et de condensation par l'aménagement d'une chambre receveuse de liquide, ledit liquide étant le premier à subir l'évaporation de part sa situation à l'extérieur de la plaque. La chambre receveuse de liquide est constituée d'une multitude de chambres entourant la matrice de puits et se prolongeant entre les puits et sous la matrice. Cependant, ce modèle reste très complexe notamment par la multitude de chambres entourant la matrice de puits et ne permettant pas une constance au niveau de l'évaporation du liquide contenue dans lesdites chambres. De plus, la complexité au niveau de la forme des chambres entourant la matrice jusque sous et entre les puits implique une fabrication coûteuse en plusieurs morceaux.

Le brevet US 7,208,125 décrit une micro-plaque comprenant une rigole périphérique destinée à recevoir un liquide faisant office de martyr d'évaporation, ladite rigole comprenant une paroi commune avec les puits de la micro-plaque situés sur la périphérie. Ce type de microplaque n'est pas satisfaisant en ce sens que la rigole périphérique ne peut pas jouer son rôle de martyr d'évaporation efficacement du fait que la rigole et les puits périphériques partagent une paroi.

D'autres essais ont été réalisés pour tenter d'optimiser les micro-plaques avec notamment l'ajout de puits supplémentaires autour de la matrice de puits de la plaque. Cependant l'art antérieur ne propose pas de solutions de réalisation simple et surtout entièrement efficace pour lutter contre ces effets de bords.

La présente invention se propose de résoudre les problèmes d'effets de bord dans les plaques de microtitration par l'ajout d'une invagination autour de la matrice de puits. Cette invagination se présente sous la forme d'une rigole périphérique continue contenant un liquide faisant office de « martyr d'évaporation », et permettant ainsi la conservation homogène et la réactivité des matériels chimiques, biochimiques ou biologiques sous l'effet de variations thermiques ou hydriques entre l'environnement et l'intérieur d'une micro-plaque.

Les micro-plaques peuvent donc supporter des variations de températures ou des contrastes hygrométriques sans que le contenu des puits ne soit altéré grâce à la compensation qu'autorise l'évaporation du liquide de la rigole périphérique continue. Les échanges gazeux entre intérieur et extérieur de la micro-plaque sont également conservés tout en maintenant la température et l'humidité dans la micro-plaque.

Le liquide contenu dans la rigole périphérique a donc pour but de protéger les puits périphériques en étant le premier à subir des variations thermiques et à s'évaporer. L'évaporation du liquide de la rigole créée ainsi une zone tampon d'hydratation et de compensation thermique des échanges gazeux entre intérieur et extérieur de la plaque recouverclée limitant fortement l'évaporation et l'altération des échantillons des puits. Les températures du milieu extérieur s'appliquent d'abord sur l'extérieur de la micro-plaque. Le liquide de la rigole sera donc le premier à s'évaporer lors de l'adaptation de la micro-plaque aux conditions d'expérimentation.

La présente invention a donc pour objet une micro-plaque comprenant une base présentant une matrice de puits et une invagination destinée à recevoir un liquide faisant office de martyr d'évaporation, ladite micro-plaque étant caractérisée en ce que ladite invagination consiste en une rigole périphérique continue.

Un avantage de l'invention est que la microplaque décrite répond aux spécifications techniques du format de microplaue défini par la SBS (d'après la nomenclature anglaise *« Society for Biomolecular Science* ») et par l'ANSI (d'après la nomenclature anglaise « *America National Standards Institute »).*

La micro-plaque selon l'invention comprend notamment une base autour d'une matrice de puits. La base est constituée de parois servant de support à la micro-plaque. Elle comprend une paroi interne, appelée « paroi interne de la base », également appelée « paroi interne de la micro-plaque » et une paroi externe nommée de manière identique « paroi externe de la base » ou « paroi externe de la micro-plaque » étant donné que les parois internes et externes de la micro-plaque correspondent à celle de la base.

Les micro-plaques comportent également au niveau des parois de la micro-plaque un décrochement. Ledit décrochement se présente de l'intérieur vers l'extérieur, formant ainsi un rebord sur la paroi externe de la micro-plaque. Le couvercle apposé sur la micro-plaque est donc maintenu grâce à ce rebord formé par le décrochement interne vers l'extérieur.

De manière standard, la micro-plaque comprend en outre une matrice de puits. Cette matrice peut être constituée d'un nombre variable de puits, destinés à recevoir autant d'échantillons à analyser. Le nombre de puits peut donc varier, mais la micro-plaque présente toujours le même schéma d'alignements desdits puits.

On considère que la paroi externe de la matrice de puits correspond à la paroi externe de la rangée de puits périphériques.

La constitution des micro-plaques standards utilisées pour la réalisation d'analyses biologiques ou biochimiques est largement connue de l'homme de l'art, et ne sera donc pas détaillée plus en détails.

Le terme « invagination » est défini comme un repliement de forme concave dans la micro-plaque consistant en une rigole périphérique continue destinée à recevoir un liquide faisant office de « martyr d'évaporation ».

Il est entendu par « liquide faisant office de martyr d'évaporation » un liquide qui, soumis à certaines contraintes pourra s'évaporer « par substitution » aux échantillons présents dans les puits périphériques de la micro-plaque comme expliqué ci-dessus.

A titre d'exemple non limitatif de « liquide faisant office de martyr d'évaporation », peuvent être mentionnés, l'eau ou tout type de solution aqueuse dont les solutions physiologiques ou solutions tampons, les sérums et milieux de cultures ainsi que les solutions de cryo-préservations. De façon équivalente, et par extension peut être mentionné comme liquide faisant office de martyr d'évaporation toute solution liquide ou pouvant se gélifier après distributions qui libère le même type de vapeurs que les échantillons contenus dans les puits. L'exemple type étant le solvant correspondant au solvant dans lequel les échantillons ou réactifs contenus dans les puits de ladite plaque sont solubilisés.

Par la suite, à moins que ne soit stipulé l'inverse, les expressions « invagination » ou « rigole périphérique continue » pourront être utilisés indifféremment.

Le liquide contenu dans la rigole périphérique continue pourra également être appelé en termes égaux « liquide faisant office de martyr d'évaporation », « liquide faisant office de rideau d'évaporation », « liquide de la rigole », « liquide de l'invagination », « liquide périphérique » ou encore « liquide d'évaporation ».

La continuité de la rigole est l'une des caractéristiques principales de la présente invention. Elle assure une uniformité totale en termes de température et d'évaporation du liquide en tout point de la rigole, le liquide étant reparti au sein d'une même rigole autour de la micro-plaque. La continuité de ladite rigole permet de ce fait une uniformisation des conditions thermiques du liquide. Cela aboutit alors à une évaporation du liquide de la rigole, protégeant alors les puits périphériques de la matrice.

Selon l'invention, la rigole périphérique continue dispose de parois propres. Il est entendu par « bords propres » ou « parois propres » de la rigole des parois que ladite rigole ne partagerait avec aucun autre élément constituant le dispositif de l'invention.

En d'autres termes, l'invention a pour objet une micro-plaque comprenant une base présentant une matrice de puits et une invagination destinée à recevoir un liquide faisant office de martyr d'évaporation, ladite micro-plaque étant caractérisée en ce que ladite invagination consiste en une rigole périphérique continue et dispose de parois propres.

La paroi externe de la rigole est donc juxtaposée à la paroi externe de la base, et la paroi interne de la rigole juxtaposée à la paroi externe de la matrice de puits. Les parois propres de la rigole permettent à ladite rigole de subir directement et de manière uniforme les conditions de températures appliquées à la micro-plaque.

La surface inférieure de la micro-plaque en contact avec le support sur laquelle elle est maintenue n'est pas uniformément plate. Comme il l'est expliqué plus haut, la base est constitué de parois comprenant un décrochement vers l'extérieur de la micro-plaque. Les puits de la micro-plaque n'atteignent pas ledit décrochement, formant ainsi un espace vide sous le fond des puits. Cet espace permet la circulation de l'air du milieu sous les puits de la matrice. L'air circulant sous les puits et la rigole permet l'application des températures d'expérimentation directement sous le dispositif comprenant le liquide périphérique et les échantillons dans les puits. L'existence de parois propres pour la rigole périphérique permet donc au liquide de ladite rigole de subir plus rapidement les températures, et ainsi de s'évaporer plus rapidement.

Ces parois uniques représentent également un avantage en terme de fabrication de la micro-plaque. En effet, il est préférable que la rigole soit isolée des puits périphériques pour éviter les contraintes mécaniques lors du démoulage et du refroidissement pendant la fabrication du dispositif.

Selon un mode préféré de l'invention, la micro-plaque est caractérisée en ce que la profondeur P de ladite invagination est inférieure ou égale à la profondeur p des puits de la matrice.

Afin que le liquide contenu dans la rigole soit bien le premier liquide à pouvoir s'évaporer sous l'effet des températures appliquées à la micro-plaque, la profondeur de la rigole ne pourra excéder celle de la matrice de puits. Il est en effet nécessaire que la surface du liquide d'évaporation soit plus haute que celle des échantillons dans les puits. Ainsi, le liquide d'évaporation pourra être le premier à s'évaporer au contact des conditions environnementales de l'air circulant sous le couvercle.

Le liquide de la rigole s'évapore sous l'effet des températures appliquées à la micro-plaque. Cette évaporation sous le couvercle de la micro-plaque permet, en plus de maintenir le contenu des puits de la matrice intacts, de stabiliser les conditions thermiques sous le couvercle au dessus des puits de la matrice.

Selon encore un autre mode de réalisation, la micro-plaque selon l'invention est caractérisée en ce que la profondeur P de ladite invagination est inférieure ou égale à la hauteur H de la portion de paroi externe de la plaque allant du haut de la plaque jusqu'au décrochement de la paroi interne de la base.

Cette réalisation de l'invention présente un avantage en terme de stockage des micro-plaques vides. En effet, au niveau industriel, les micro-plaques sont empilées les unes au dessus des autres. L'espace vide sous les puits de la matrice permet ainsi la possibilité d'empiler les micro-plaques pour un minimum d'espace de stockage. La rigole ne devra donc pas dépasser le décrochement du bord interne de la micro-plaque afin de permettre l'empilement des micro-plaques pour un volume de stockage réduit.

Selon une forme d'exécution préférée, la micro-plaque selon l'invention est caractérisée en ce que l'épaisseur du fond de ladite invagination est identique à l'épaisseur du fond des puits de la matrice.

Pour que les conditions thermiques s'appliquent de manière identique à la rigole et aux puits de la matrice, il est nécessaire que les fonds de ladite rigole et desdits puits soient constitués de manière similaire en terme d'épaisseur.

Cela permet également une économie de plastique et de poids pour le transport et l'emballage.

La forme de la rigole périphérique continue est une caractéristique importante de l'invention. En effet, elle est responsable de la bonne évaporation du liquide qu'elle contient pour protéger les échantillons à l'intérieur des puits de la matrice des effets de température et d'hygrométrie appliqués à la micro-plaque.

Selon une forme d'exécution préférée de l'invention, l'invagination formant la rigole présente au moins une paroi non rectiligne dont la forme vient épouser le rebord circulaire de la rangée de puits périphériques de la matrice.

De manière préférée, au moins une paroi de cette rigole est non rectiligne et constitue la paroi interne, c'est-à-dire celle à proximité de la rangée externe des puits. Cependant, pour toute raison technique, il pourra s'agir de l'autre paroi, à savoir celle à proximité des parois externes de la micro-plaque.

Cette forme particulière d'au moins une paroi de la rigole périphérique continue a pour conséquence l'allongement de ladite rigole. Le liquide d'évaporation s'étend alors sur un périmètre plus important autour de la matrice de puits. En plus d'épouser la forme circulaire des puits de la matrice, la paroi non rectiligne de la rigole est située à distance constante desdits puits. La proximité entre la rigole et les puits périphériques joue un rôle dans l'évaporation du liquide de la rigole seulement et non des échantillons dans les puits périphériques qui sont alors protégés par ladite rigole. La forme non rectiligne de la paroi de la rigole épousant le rebord circulaire des puits périphériques de la matrice a pour conséquence d'augmenter au maximum la longueur de la rigole périphérique continue, et donc ainsi le volume de liquide d'évaporation.

La forme serpentante de la paroi non rectiligne de la rigole joue également un rôle au niveau de la stabilité du liquide d'évaporation. En effet, le déplacement de la micro-plaque peut entraîner des mouvements de liquide, notamment dans la rigole continue. Ces mouvement sont dus au fait que le liquide périphérique présente plus de mobilité du fait de la continuité longitudinale de la rigole que les échantillons dans les puits de la micro-plaque. Ainsi, la forme non rectiligne d'au moins une paroi de la rigole peut casser les ondes de chocs dans le liquide de la rigole, en limitant les mouvements dudit liquide.

Selon une autre forme de réalisation également préférée, la micro-plaque selon l'invention est caractérisée en ce que ladite rigole périphérique continue présente ses deux parois non rectilignes dont la forme vient épouser le rebord circulaire de la rangée de puits périphériques de la matrice.

Les mêmes avantages observés par la présence d'une seule paroi de la rigole non rectiligne sont également rencontrés avec la présence de deux parois non rectilignes. La surface de la rigole est d'autant plus agrandie et les ondes de chocs dues à la mobilité du liquide dans la rigole sont d'autant plus contrôlées et limitées par la présence de ces deux parois non rectilignes.

Selon un mode particulier de réalisation de l'invention, il peut être prévu au sein de la rigole périphérique, selon un axe perpendiculaire, des parois formant demi chicane. De telles parois ne devront pas traverser de part en part la rigole périphérique afin que cette dernière garde sa forme continue pour pouvoir assurer son remplissage facilement en une seule opération. De telles chicanes auront pour effet de casser les ondes de chocs dans le liquide et d'en augmenter la tension de surface globale. L'utilisation de demi chicane pourra remplacer la forme serpentante de la rigole ou pourra venir en complément.

Un autre avantage de l'invention, directement lié à la continuité de la rigole périphérique, réside dans le fait que l'invagination formant rigole présente au moins un évasement faisant office de point de remplissage de ladite rigole.

Il est ici mentionné que l'expression « évasement » pourra être remplacé par tout terme synonyme dans la présente description, notamment par l'expression « élargissement ».

La continuité de la rigole, caractéristique principale de l'invention, présente l'avantage que le liquide « martyr d'évaporation » peut être administré dans la rigole en une seule distribution. La largeur de la rigole ne permettant pas forcément un remplissage aisé, il a été rajouté à la rigole un évasement de taille relativement réduite permettant ledit remplissage de la rigole avec le liquide d'évaporation.

De manière privilégiée, ledit évasement est localisé au niveau d'un angle de la micro-plaque. Cet emplacement permet ainsi de maintenir la continuité de la rigole tout autour des puits en périphérie de la micro-plaque. La localisation de l'évasement au niveau de l'angle de la micro-plaque permet ainsi de réduire les différences de distances pouvant se présenter avec les puits proches de l'évasement comparé à celui des puits au niveau de la rigole.

Selon un mode de réalisation préféré, la largeur de la rigole périphérique continue est comprise entre 2 millimètres et 2,5 millimètres au niveau du diamètre des puits et entre 5 et 6 millimètres au niveau de la jonction entre les puits.

Cette largeur a été calculé de manière à être la mieux adaptée aux dispositifs de micro-plaque et machines existant et permet, entre autre, d'utiliser des couvercles standards. Toutefois, il est bien évident que cette largeur pourra être modifiée par l'homme de l'art en fonction des évolutions des différents appareillages.

En pratique, la coupe transversale de la rigole périphérique continue peut présenter diverses formes comme, par exemple, une forme de cône, de trapèze rectangle inversé ou encore de rectangle. De manière générale, il est préférée une forme légèrement trapézoïdale pour permettre un angle de dépouille suffisant au moulage et pour que le liquide fuse tout autour de la rigole lors du remplissage sans piéger de bulles ou provoquer un débordement alors que le liquide n'a pas encore remplit toute la rigole. Il ne faut pas qu'elle soit non plus trop conique pour que la largeur de liquide dans la rigole ne diminue pas trop rapidement avec l'abaissement de la hauteur de liquide en son sein. L'effet protecteur diminuerait alors trop avec le temps au moment où le bénéfice hydratant est le plus nécessaire.

De manière préférée, la micro-plaque selon l'invention comprend un espace au niveau de la périphérie extérieure de la micro-plaque, ledit espace étant suffisamment large pour permettre le marquage alphanumérique dudit dispositif.

Compte tenu du nombre de puits de certaines micro-plaques, le marquage alphanumérique desdits puits facilite l'utilisation des micro-plaques par un homme du métier. Le marquage peut s'effectuer sur le bord de la micro-plaque sur la base. Il permet une reconnaissance par une lettre ou un chiffre de manière horizontale et verticale. Il est donc important d'inclure cet espace permettant le marquage alphanumérique des puits dans la micro-plaque de l'invention.

De manière standard, la micro-plaque selon l'invention peut être constituée de 6, 12, 24, 48, 96, 384 ou 1536 puits.

Il existe à l'heure actuelle une importante variété de micro-plaques pour la réalisation de synthèses, réactions, stockages ou analyses chimiques, biologiques ou biochimiques ou encore la culture de cellules. Ainsi, le modèle de dispositif présenté dans cette demande de brevet est applicable à tout type de micro-plaque standard utilisée en laboratoires d'analyses, de chimie, de biologie ou biochimie.

Selon un autre aspect, l'invention concerne un dispositif comprenant une micro-plaque telle que décrit plus haut et un couvercle standard conçu de manière à recouvrir intégralement mais non hermétiquement la matrice de puits et la rigole d'évaporation de ladite micro-plaque.

Le couvercle est notamment utilisé dans le but de protéger les échantillons présents dans les puits de la micro-plaque. Ledit couvercle est un couvercle standard utilisé dans de nombreux modèles de micro-plaques. Il permet le maintien et l'équilibrage des échanges gazeux au sein de la micro-plaque au dessus des puits de la matrice et de la rigole tout en autorisant une légère ventilation avec l'environnement de la micro-plaque. Une telle ventilation est en effet nécessaire au maintient de la viabilité et/ou fonctionnalité de certains matériels contenus dans les puits. En ce sens, la présente invention utilisée conjointement avec un couvercle standard permet une telle ventilation sans pour autant aboutir à une évaporation du contenue des puits périphériques en vertu des bénéfices apportés par la rigole.

Il se pose de manière simple sur la micro-plaque au niveau du rebord formé par le décrochement interne de la base. Ledit décrochement de la base sur l'extérieur de la micro-plaque permet ainsi le calage du couvercle sur la micro-plaque.

Selon un autre aspect, l'invention concerne un dispositif comprenant une micro-plaque telle que décrit plus haut et un couvercle spécifique conçu de manière à recouvrir intégralement et hermétiquement la matrice de puits et la rigole d'évaporation de ladite micro-plaque. Ce dispositif permet alors d'équilibrer les échanges gazeux au dessus des puits de la matrice et de la rigole de façon définitive en vue d' un stockage à long terme des échantillons contenus dans les puits.

Selon encore un autre aspect, l'invention vise un procédé de fabrication du dispositif mentionné ci-dessus caractérisé en ce qu'il comprend une première étape de fabrication de la micro-plaque selon l'invention et une deuxième étape de fabrication du couvercle.

La fabrication de la micro-plaque en seulement deux étapes présente un avantage industriel non négligeable pour la simplicité de fabrication du dispositif de l'invention. La première étape consiste donc en la fabrication de la micro-plaque elle-même, constituée de la base, la rigole périphérique continue ainsi que la matrice de puits. Elle est donc réalisée en un seul morceau, caractérisant sa simplicité de fabrication. La deuxième étape de fabrication concerne la fabrication du couvercle de la micro-plaque, étape ne présentant aucune difficulté de fabrication étant donné que le couvercle utilisé pour la micro-plaque de l'invention est un couvercle standard.

Enfin, selon un dernier aspect, l'invention couvre également l'utilisation d'une micro-plaque et/ou d'un dispositif obtenu par la mise en oeuvre du procédé selon l'invention pour la culture et/ou primo-culture de cellules eucaryotes et/ou procaryotes, les cultures de tissus et/ou proto organes, ainsi que le stockage, les réactions et analyses de matériels, et réactifs chimiques, biochimiques et/ou biologiques.

Plus particulièrement, et de manière avantageuse, l'invention vise l'utilisation visée ci-dessus caractérisée en ce que ladite invagination formant rigole est remplie d'un liquide en une seule distribution.

Le remplissage de la rigole périphérique continue en une seule étape de distribution est possible grâce à la continuité de la rigole. Cette utilisation présente donc une certaine praticité pour l'Homme de l'art qui n'a pas à réaliser plusieurs remplissages autour de la matrice de puits.

L'invention sera décrite plus en détails dans les exemples ci-après faisant référence aux figures suivantes :
Figure 1 : Vue de dessus d'une micro-plaque 96 puits selon l'invention,
Figure 2 : Vue en coupe transversale d'un premier mode de réalisation au niveau du diamètre des puits (A) et au niveau de la jonction entre les puits (B),
Figure 3 (A à F) : diagrammes illustrant la mesure de viabilité cellulaire après 3 ou 4 jours d'incubation dans des micro-plaques 96 puits commerciales versus des micro-plaques selon l'invention.

### Exemple 1

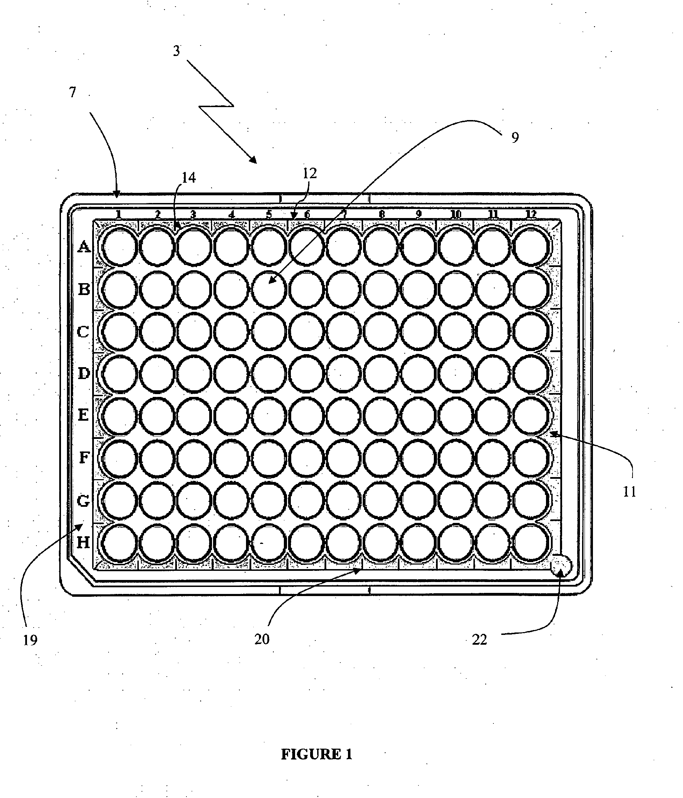

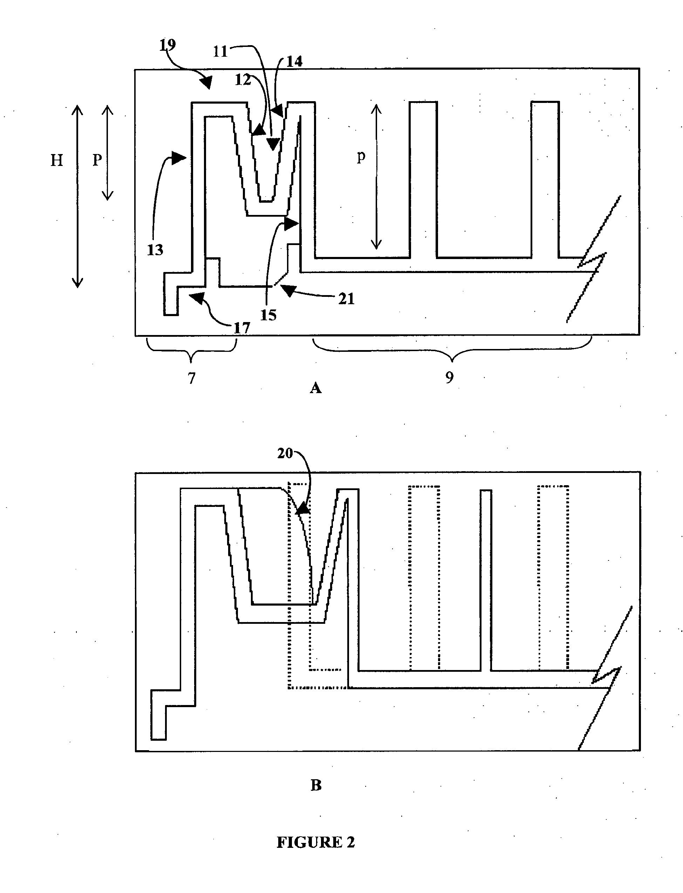

En se référant aux figures 1 et 2, le dispositif est constitué d'une micro-plaque 3 et d'un couvercle 5 (non représenté). La micro-plaque 3 comprend une base 7 entourant une matrice de puits 9. La micro-plaque 3 comprend également une invagination 11 destinée à recevoir un liquide faisant office de « martyr d'évaporation ». Comme cela ressort clairement de la Figure 1, ladite invagination 11 de la micro-plaque 3 consiste en une rigole périphérique continue. La rigole périphérique continue 11 est située entre la paroi externe 13 de la base 7 et la paroi externe 15 de la matrice de puits 9. Selon un modèle préféré de l'invention illustré sur la figure 2, la rigole 11 dispose de parois propres, soit une paroi externe 12 et une paroi interne 14. La profondeur P de la rigole périphérique continue 11 est inférieure ou égale à la profondeur p des puits de la matrice. La profondeur P de la rigole périphérique continue 11 ne dépasse pas la hauteur H du décrochement 17 de la paroi interne 13 de la base 7. La rigole périphérique continue 11 présente au moins une paroi non rectiligne 14 dont la forme vient épouser le rebord circulaire de chaque part de la rangée de puits périphériques de la matrice 9. La rigole périphérique continue 11 peut également présenter deux parois non rectilignes 12 et 14 dont la forme vient épouser le rebord circulaire de chaque part de la rangée de puits périphériques de la matrice 9 (forme d'exécution non représentée).

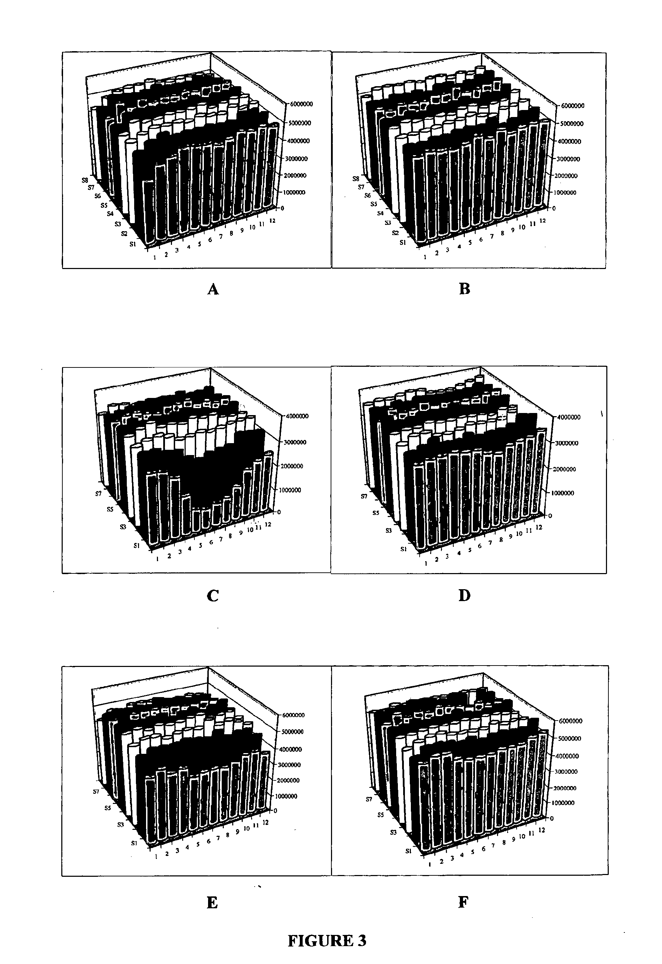

La rigole 11 présente en son sein des demies chicanes 20 localisées dans la rigole au niveau de la jonction entre deux puits sur les modèles préférés des figures 1 à 3.

La micro-plaque décrite selon la présente invention peut présenter, en outre, des chicanes de renforts 21 entre la paroi externe 13 de la micro-plaque et la paroi externe 15 de la matrice de puits 9.

La rigole périphérique continue 11 présente au moins un élargissement ou évasement 22 faisant office de point de remplissage de ladite rigole 11. L'élargissement 22 est localisé au niveau d'un angle de la micro-plaque 3. La plaque 3 comprend un espace 19 sur l'extérieur de la base 7 permettant le marquage alphanumérique du dispositif.

### Exemple 2

Les exemples décris ici sont réalisés sur micro-plaques 96 puits ensemencées par des lignées cellulaires à un nombre constant de cellules par puits.

Les résultats correspondent à une mesure de viabilité cellulaire après 3 ou 4 jours d'incubation dans des micro-plaques 96 puits commerciales versus une incubation identique dans des micro-plaques dont les bords ont été flanqués de solution aqueuse saline.

*Expérience 1 :* représentation sous forme d'histogramme 3D des résultats de mesure de viabilité cellulaire de la lignée NCI-460 après 72 h d'incubation dans un incubateur 1 .Chacune des 96 barres représente respectivement la valeur de signal proportionnel à la viabilité correspondante a chaque puits d'une micro-plaque 96 puits standard pour l'histogramme A et, en B d'une micro-plaque selon l'invention.

*Expérience 2 :* représentation sous forme d'histogramme 3D des résultats de mesure de viabilité cellulaire de la lignée WM 266-4 après 72 h d'incubation dans un incubateur 2 .Chacune des 96 barres représente respectivement la valeur de signal proportionnel à la viabilité correspondante a chaque puits d'une micro-plaque 96 puits standard pour l'histogramme C et, en D d'une micro-plaque selon l'invention.

*Expérience 3 :* représentation sous forme d'histogramme 3D des résultats de mesure de viabilité cellulaire de la lignée WM 266-4 après 96 h d'incubation dans un incubateur 2 .Chacune des 96 barres représente respectivement la valeur de signal proportionnel à la viabilité correspondante a chaque puits d'une micro-plaque 96 puits standard pour l'histogramme E et en F d'une micro-plaque selon l'invention.

## Revendications

1. Micro-plaque (3) comprenant une base (7) présentant une matrice de puits (9) et une invagination (11) destinée à recevoir un liquide faisant office de martyr d'évaporation, ladite micro-plaque (3) étant **caractérisée en ce que** ladite invagination (11) consiste en une rigole périphérique continue et dispose de parois propres.

2. Micro-plaque selon la revendication 1, **caractérisé en ce que** ladite invagination (11) est située entre la paroi externe (13) de la base (7) et la paroi externe (15) de la matrice de puits (9).

3. Micro-plaque selon l'une des revendications 1 ou 2, **caractérisée en ce que** la profondeur P de ladite invagination (11) est inférieure ou égale à la profondeur p des puits de la matrice (9).

4. Micro-plaque selon l'une des revendications 1 à 3, **caractérisée en ce que** la profondeur P de ladite invagination (11) est inférieure ou égale à la hauteur H de la portion de paroi externe (13) de la plaque (3) allant du haut de la plaque (3) jusqu'au décrochement (17).

5. Micro-plaque selon l'une des revendications 1 à 4, **caractérisée en ce que** l'épaisseur du fond de ladite invagination (11) est identique à l'épaisseur du fond des puits de la matrice (9).

6. Micro-plaque selon l'une des revendications 1 à 5, **caractérisée en ce que** ladite invagination (11) présente au moins une paroi non rectiligne (14) dont la forme vient épouser le rebord circulaire de la rangée de puits périphériques de la matrice (9).

7. Micro-plaque selon la revendication 6, **caractérisée en ce que** ladite rigole périphérique continue (11) présente ses deux parois non rectilignes (12) et (14) dont la forme vient épouser le rebord circulaire de la rangée de puits périphériques de la matrice (9).

8. Micro-plaque selon l'une des revendications 1 à 7 **caractérisé en ce que** ladite invagination (11) présente au moins un évasement (22) faisant office de point de remplissage de ladite rigole.

9. Micro-plaque selon l'une des revendications 1 à 8, **caractérisée en ce que** la largeur de ladite invagination (11) est comprise entre 2 millimètres et 2,5 millimètres dans l'axe du diamètre des puits et 5 à 6 millimètres dans l'axe de la jonction entre deux puits.

10. Dispositif comprenant une micro-plaque (3) selon l'une quelconque des revendications 1 à 9 et un couvercle (5) conçu de manière à recouvrir intégralement la matrice de puits (9) et la rigole (11) de ladite micro-plaque (3).

11. Procédé de fabrication du dispositif selon la revendication 10 **caractérisé en ce qu'**il comprend une première étape de fabrication de la micro-plaque (3) selon l'invention et une deuxième étape de fabrication du couvercle (5).

12. Utilisation d'une micro-plaque selon l'une quelconque des revendications 1 à 9 et/ou d'un dispositif selon la revendication 10 et/ou d'un dispositif obtenu par la mise en oeuvre du procédé selon la revendication 11 pour la culture et/ou primo-culture de cellules eucaryotes et/ou procaryotes, les cultures de tissus et/ou proto organes, ainsi que le stockage, les réactions et analyses de matériels, et réactifs chimiques, biochimiques et/ou biologiques.

13. Utilisation selon la revendication 12, caractérisée eh ce que ladite invagination (11) est remplie d'un liquide en une seule distribution.

## Patentansprüche

1. Mikroplatte (3), welche eine Basis (7) umfasst, die eine Matrix aus Vertiefungen (9) und eine Einstülpung (11) aufweist, die dazu bestimmt ist, eine Flüssigkeit aufzunehmen, die zur Verdampfung bestimmt ist, wobei die Mikroplatte (3) **dadurch gekennzeichnet ist, dass** die Einstülpung (11) aus einer kontinuierlichen peripheren Rinne besteht und über eigene Wände verfügt.

2. Mikroplatte gemäß Anspruch 1, **dadurch gekennzeichnet, dass** sich die Einstülpung (11) zwischen der Außenwand (13) der Basis (7) und der Außenwand (15) der Matrix aus Vertiefungen (9) befindet.

3. Mikroplatte gemäß einem der Ansprüche 1 oder 2, **dadurch gekennzeichnet, dass** die Tiefe P der Einstülpung (11) geringer als oder gleich der Tiefe p der Vertiefungen der Matrix (9) ist.

4. Mikroplatte gemäß einem der Ansprüche 1 bis 3, **dadurch gekennzeichnet, dass** die Tiefe P der Einstülpung (11) geringer als oder gleich der Höhe H des Abschnitts der Außenwand (13) der Platte (3), der von der Oberseite der Platte (3) bis zum Absatz (17) verläuft, ist.

5. Mikroplatte gemäß einem der Ansprüche 1 bis 4, **dadurch gekennzeichnet, dass** die Dicke des Bodens der Einstülpung (11) iden-tisch mit der Dicke des Bodens der Vertiefungen der Matrix (9) ist.

6. Mikroplatte gemäß einem der Ansprüche 1 bis 5, **dadurch gekennzeichnet, dass** die Einstülpung (11) wenigstens eine nicht gerade Seitenwand (14) aufweist, deren Form sich an den kreisförmigen Rand der Reihe von peripheren Vertiefungen der Matrix (9) anschmiegt.

7. Mikroplatte gemäß Anspruch 6, **dadurch gekennzeichnet, dass** die kontinuierliche periphere Rinne (11) zwei nicht gerade Seiten-wände (12) und (14) aufweist, deren Form sich an den kreisförmigen Rand der Reihe von peripheren Vertiefungen der Matrix (9) an-schmiegt.

8. Mikroplatte gemäß einem der Ansprüche 1 bis 7, **dadurch gekennzeichnet, dass** die Einstülpung (11) wenigstens eine Ausbau-chung (22) aufweist, die als Füllanschluss der Rinne dient.

9. Mikroplatte gemäß einem der Ansprüche 1 bis 8, **dadurch gekennzeichnet, dass** die Breite der Einstülpung (11) 2 Millimeter bis 2,5 Millimeter auf Höhe des Durchmessers der Vertiefungen und 5 bis 6 Millimeter auf Höhe der Verbindungsstelle zwischen zwei Vertiefungen beträgt.

10. Vorrichtung, welche eine Mikroplatte (3) gemäß irgendeinem der Ansprüche 1 bis 9 und einen Deckel (5) umfasst, der derart konzipiert ist, dass er die Matrix aus Vertiefungen (9) und die Rinne (11) der Mikroplatte (3) vollumfänglich abdeckt.

11. Verfahren zur Herstellung einer Vorrichtung gemäß Anspruch 10, **dadurch gekennzeichnet, dass** es einen ersten Schritt der Herstellung der erfindungsgemäßen Mikroplatte (3) und einen zweiten Schritt der Herstellung des Deckels (5) umfasst.

12. Verwendung einer Mikroplatte gemäß irgendeinem der Ansprüche 1 bis 9 und/oder einer Vorrichtung gemäß Anspruch 10 und/oder einer Vorrichtung, die durch Ausführung des Verfahrens ge-mäß Anspruch 11 erhalten wird, für die Kultivierung und/oder Erstkulti-vierung von eukaryotischen und/oder prokaryotischen Zellen, die Kulti-vierung von Geweben und/oder Protoorganen sowie für die Lagerung, Reaktionen und Analysen von Stoffen und chemischen, biochemischen und/oder biologischen Reagenzien.

13. Verwendung gemäß Anspruch 12, **dadurch gekennzeichnet, dass** die Einstülpung (11) in einer einzigen Verteilung mit einer Flüssigkeit ausgefüllt wird.

## Claims

1. A microplate (3) comprising a base (7) having a matrix of wells (9) and an invagination (11), arranged to receive a liquid which acts as an evaporation martyr, said microplate (3) being **characterized in that** said invagination (11) consists of a continuous peripheral channel with walls of its own.

2. The microplate according to claim 1, **characterized in that** said invagination (11) is located between the outer wall (13) of the base (7) and the outer wall (15) of the matrix of wells (9).

3. The microplate according to one of claims 1 or 2, **characterized in that** the depth P of said invagination (11) is less than or equal to the depth p of the wells of the matrix (9).

4. The microplate according to one of claims 1 to 3, **characterized in that** the depth P of said invagination (11) is less than or equal to the height H of the portion of the outer wall (13) of the plate (3) which extends from the top of the plate (3) to the jutting-out portion (17).

5. The microplate according to one of claims 1 to 4, **characterized in that** the thickness of the bottom of said invagination (11) is the same as the thickness of the bottom of the wells of the matrix (9).

6. The microplate according to one of claims 1 to 5, **characterized in that** said invagination (11) has at least one non-rectilinear wall (14) whose shape matches the circular edge of the row of peripheral wells of the matrix (9).

7. The microplate according to claim 6, **characterized in that** said continuous peripheral channel (11) has two non-rectilinear walls (12) and (14) whose shape matches the circular edge of the row of peripheral wells of the matrix (9)

8. The microplate according to one of claims 1 to 7 **characterized in that** said invagination (11) has at least one bulge (22) acting as a filling point of said channel.

9. The microplate according to one of claims 1 to 8, **characterized in that** the width of said invagination (11) is between 2 millimeters and 2.5 millimeters in the axis of the diameter of the wells and 5 to 6 millimeters in the axis of the junction between two wells.

10. A device comprising a microplate (3) according to any one of claims 1 à 9 and a cover (5) arranged to fully cover the matrix of wells (9) and the channel (11) of said microplate (3).

11. A method of manufacturing the device according to claim 10 **characterized in that** it comprises a first step of manufacturing the microplate (3) of the invention and a second step of manufacturing the cover (5).

12. Use of a microplate according to any one of claims 1 to 9 and/or of a device according to claim 10 and/or of a device obtained by carrying out the method according to claim 11 for the culture and/or primary-culture of eukaryotic and/or prokaryotic cells, tissue and/or proto-organ cultures, and also storage, reactions and analysis of materials, and chemical, biochemical and/or biological reagents.

13. Use according to claim 12, **characterized in that** said invagination (11) is filled with a liquid in a single introduction.
